# EUROPEAN PATENT APPLICATION

(11) **EP 4 529 847 A2**
(43) Date of publication of application: **02.04.2025**
(21) Application number: 24203422.1
(22) Date of filing: 29.09.2024
(51) Int. Cl.: A61B 6/00

(54) **IMAGE ACQUISITION APPARATUS**

(30) Priority: 29.09.2023 IT 202300020106
(71) Applicant: ATS Srl -Società con socio unico, 24060 Torre de' Roveri (BG) (IT)
(72) Inventor: MOIOLI, Mauro, Bergamo (BG) (IT); GERINI, Mattia, Sanremo (IM) (IT)
(74) Representative: Karaghiosoff, Giorgio Alessandro

(57) **Abstract**

An image acquisition apparatus with an arched or C-shaped support arm, wherein the C-shaped arm includes a body made from a composite profile, specifically a material that includes one or more reinforcing fibers, preferably mineral fibers, particularly carbon fibers, embedded in a matrix incorporating these fibers. Along the longitudinal extension of the arm, on its circumferential, axial, and radial surfaces-relative to the arm's curvature axis-one or more rolling or sliding tracks are arranged. These tracks are composed of hard-profiled materials, especially metal, applied to the surfaces of the profile.

According to an additional feature, which may be implemented independently or in combination with the previous one, the C-arm is designed for radiographic imaging apparatuses, supporting at its ends an X-ray source and a detector, which face each other. At least the source and/or the detector are translatable in a direction to move closer together or further apart. A counterweight mass is associated with the support end of the source and/or the detector to compensate for any shift in the center of gravity due to the translation of the source and/or the detector, with the counterweight also being translatable in a direction parallel to the translation path of the source and/or detector.

## Description

The present invention relates to an image acquisition apparatus, specifically a mobile device for capturing diagnostic images.

More specifically, the invention relates to a mobile image acquisition apparatus with an arcuate or C-shaped support arm for one or more image acquisition units according to one or more scanning techniques.

A specific embodiment refers to the apparatuses of the aforementioned type intended for radiological image acquisition, comprising a base and a slide support for an arcuate arm, preferably shaped as a circular sector, which arcuate arm is coupled or integrally formed with slide guides along its circumferential extension, which slide guides cooperate with rotatable supports mounted rotatably within said slide support of the said arcuate arm, with an optional motorised drive element for the said sliding movement being provided.

In one embodiment, said arcuate arm is supported to rotate around a rotational axis that is radial relative to it and/or tangentially intersecting the line joining the two ends of the arc along which the arm itself extends.

When the intended scan is radiographic, this rotation enables for example image acquisition with the technology known as CBCT (Cone Beam Computed Tomography).

When the image acquisition method is radiographic, typically, the arcuate arm carries, at angularly spaced points, preferably located at a terminal section of the two opposing ends of the arcuate arm, respectively an X-ray source and an X-ray detector arranged opposite each other, while the intermediate area between said source and said detector, encompassed by the arcuate arm, constitutes a positioning space for a body under examination or other target to undergo radiographic examination.

The above-described construction of arcuate arm machines, for both radiographic image acquisition and other image acquisition systems or methods, has been known for a long time in the technical field, and in the state of the art there are various solutions concerning the design of the arcuate arm, the construction of the slide guides and slide support, as well as the mounting of the said scanning and signal acquisition units for the body under examination, from which images are processed, in radiographic applications represented by the X-ray source and detector

There are variants where the guides associated with the arcuate arm are separate parts attachable to the walls of said arm and also variants where the guides and the arcuate arm are integrally formed from a single profile featuring a cross-section comprising a structural part constituting the arm body and extensions projecting from one or more perimeter surfaces of said body, such as in the form of ribs or fins intended to form the slide guides for sliding elements like rolling elements, for example track rollers or similar, or sliding elements, like shoes or similar

The body of the arcuate arm may also not be a single piece but formed from two or more profiles sectioned along planes that are coaxial with the arcuate shape of the arm, which are joined together in a stable or removable way to form the arm body. This solution is possible both when the guides are integrally formed with the arcuate or "C"-shaped arm body and when they are separate parts fixed to the arcuate or "C"-shaped arm body.

Image acquisition machines for target bodies, and specifically but not limited to, radiographic machines of the type described, are used for acquiring images of a target body during activities performed on said target body, which is positioned on a support designed according to the requirements of the activities to be carried out on said target body. These machines find applications, for example, in the medical or veterinary fields, in surgical procedures, or in diagnostic imaging acquisitions during therapeutic follow-up activities or forensic examinations to monitor patient condition progress or in diagnostic imaging of bedridden patients. Beyond medical or veterinary applications, image acquisitions can also be performed in materials inspection and/or analysis of various other object types.

The imaging apparatuses intended for these applications feature a base, typically mounted on a carriage or configured as a carriage, to be moved to the position of the target body to be examined, such as a patient, with the said target body, i.e. said patient, in the correct imaging position relative to the scanning and image acquisition unit. In radiographic applications, the target body is typically positioned between the source and the detector.

The radial width of the arcuate arm must allow the optical opening area of the image acquisition unit, for example, the X-ray source and/or detector, to be positioned at areas of interest on a body under examination, with the said area of interest to be imaged coinciding with the optical opening area of the scanning or image acquisition unit, for example, the source-detector system. In performing radiographic imaging on human and/or animal patients, this radial width must necessarily be relatively large to allow positioning of the source and detector at a predetermined distance from each other, with the patient's body and any support structure, like a table or bed, positioned intermediate between said source and said detector.

For example, during surgery, it is often necessary to acquire images at multiple time intervals to monitor the procedure's progress, requiring the apparatus to be moved repeatedly to an operational imaging position and away from the surgeon's and assistants' workspace.

The imaging machine with an arcuate or "C"-shaped arm is thus required to be as light as possible to facilitate movement, to ensure the necessary dimensions for handling the intended target bodies, such as patients in various possible conditions, and to exhibit high mechanical strength in the support and movement system of the arm, preventing dynamic or static stresses of the masses of the scanning and/or image acquisition units, like in radiographic scans, the source and detector (masses cantilevered relative to the slide support) from generating deformations in the guides and/or the slide support, thus preventing clearance from arising between the arm and the slide support.

Document EP3919001 shows a radiographic apparatus with a C-arm composed of a hybrid profile, where the C-arm is guided along its circumference on a support profile within a slide support unit. The hybrid profile comprises a lightweight arcuate profile section forming the C-arm's base body and a circular arc-shaped steel profile that acts as a guide profile with a section in a diametral plane relative to the arm's curvature axis in the shape of a cylindrical cladding sector attached to the convex side of the C-arm's base body. The said guide profile part includes sliding surfaces on the concave and convex sides that cooperate with support rollers. Along two opposing lateral ridges of the said second profile part, there are guides for lateral guide rollers.

Also document US2021/378610 describes a hybrid C-shaped profile with an arcuate shape for an X-ray device with a C-arm, said profile featuring a lightweight profile as the main body and a steel profile as the support profile for a bearing unit, the lightweight profile and steel profile connected by connection means.

Document US5425068 describes a support element for at least one heavy piece of medical equipment in a medical diagnostic station. The said support element is made of fibre-reinforced plastic and has a cross-section that is longer in one direction than the other. The fibre-reinforced plastic serves as a shell surrounding a molded element, which may be a lightweight plastic material, such that the fibre-reinforced plastic fulfils the load-bearing support functions for the element. The support element can be configured to allow adjustable movement along its length, featuring metal guides with which the casters of a carriage engage.

Document US2021145383 describes various methods and systems for a medical imaging system with a C-arm. In one embodiment, an imaging system includes a C-arm comprising an inner circumferential wall forming a first pair of grooved flanges and an outer circumferential wall forming a second pair of grooved flanges, where each of the first and second pairs of grooved flanges includes a composite material. A C-shaped portion of the C-arm may be configured to rotate isocentrically around a rotational axis.

As is evident, the arm constructed from a hybrid profile, specifically with a radially inner, lighter arcuate section and a radially outer steel section, only partially addresses the trade-off issues between mechanical strength and overall weight, as the guiding part of the arm still contains a significant amount of metal material, thus retaining considerable weight. Furthermore, this construction is complex and potentially less durable, particularly regarding the reciprocal fastening processes of the two frame parts and the durability of this fastening over time.

From a construction perspective, this solution also requires manufacturing two different arcuate-shaped parts that must be precisely formed in terms of the arcuate shape and also concerning the conforming and coincident positioning of the reciprocal fastening elements of the two parts.

The present invention aims to overcome the drawbacks of known arcuate or C-arm image acquisition machines by enabling a further optimisation of the weight, dimensions, mechanical strength, and wear resistance ratio.

The present invention solves the stated problem with an apparatus for image acquisition of a target body of the type described initially, in which said apparatus comprises a base and a slide support for an arcuate arm, preferably shaped like a circular sector, which arcuate arm is coupled or integrally formed with slide guides along its circumferential extension, which slide guides cooperate with rotatable supports mounted rotatably within said slide support of said arcuate arm, with an optional motorised drive element for the said sliding movement being provided, and wherein
the said arm is entirely made from an arcuate profile in a composite material comprising reinforcing fibres, in particular carbon fibres, and an embedding matrix of the said fibres, which profile forms the arm body and integrally features one or more guiding surfaces with an axial and/or radial orientation, that is, parallel and/or perpendicular to the arm's curvature axis and which guiding surfaces feature rolling paths for at least one corresponding roller or at least one corresponding caster, formed from continuous metallic profiles fixed at a coincident position with the paths of the peripheral contact surfaces of said rollers or casters, facing the said peripheral contact surfaces along the said guiding surfaces.

In one embodiment, the guiding surfaces are formed by a pair of fins projecting outward in a direction parallel to the arm's curvature axis, at a predetermined distance from each of the two opposite side walls of the profile, which fins are oriented in a direction parallel to the arm's curvature axis and are parallel, i.e. concentric, to each other and feature the aforementioned axial rolling paths on the mutually facing sides of said fins, being provided on each side of the arm at least one pair of rollers or casters, with the rollers of each pair being in contact with one of the two mutually facing rolling paths on the fins.

According to an additional feature, a third radial rolling path, coaxial to the axial rolling paths, is provided on each of the two opposing side walls of the arm along the band of said side walls, which is radially delimited by said projecting fins on the corresponding side, while the support is provided with at least one roller or at least one caster mounted on the support of the arm in a position to adhere respectively to one of the radial lateral rolling paths.

In one embodiment, the slide support thus comprises:
At least one pair of rollers or casters aligned at a predetermined distance from each other in a radial direction relative to the arm's curvature axis and rotatable around axes parallel to the arm's curvature axis, the pairs of said rollers or casters are provided or can be positioned at a predetermined distance from each other such that each of the rollers in the pair adheres with a predetermined pressure against the corresponding axially oriented rolling path;
and at least one roller or caster with a rotation axis oriented radially relative to the arm's curvature axis and supported in a position such that each roller or caster adheres to the radial rolling path with a predetermined pressure;
the aforementioned rollers allowing the arm to slide and keeping the arm in position with radial or axial play relative to its curvature axis, limited within predetermined tolerances.

In one embodiment, the peripheral surfaces of the rollers and/or casters with which said rollers or casters adhere to the rolling paths are preferably coated with a layer of plastic material having a predetermined elastic deformability, providing cushioned support of said rollers and/or casters against said rolling paths.

In one embodiment, the rollers or casters are constituted by bearings whose inner race is fitted onto a spindle coaxial with the rotation axis, and whose outer race adheres with its outer surface against the corresponding rolling path.

According to the additional feature described above, the radially outermost race features a plastic coating on the external circumferential mantle surface, presenting a predetermined thickness in the radial direction and predetermined characteristics of elastic deformability, in particular, elastic compressibility.

The bearings may be of any type, such as ball bearings, cylindrical roller bearings, or with elliptical or crowned rollers.

With reference to an additional characteristic that can be included in combination with any one or more of the embodiments and variants described in this document, the slide support can include two or more pairs of rollers on each side of the arcuate arm, respectively cooperating with the axial rolling paths provided on the corresponding guides and which pairs of rollers or casters are spaced at a predetermined distance from each other in the direction of the angular extension, that is, in the circumferential direction of the arcuate arm.

One embodiment provides that the rollers of each pair, which respectively cooperate with one of the two opposing axial rolling paths, are mounted on separate carriages.

In one embodiment, two or more rollers or casters cooperating with one of the two facing axial rolling paths are rotatably mounted around their axis on a carriage, the said carriage being supported to oscillate in a rocker-like manner around an axis parallel to the rotation axis of the rollers, provided in an intermediate position relative to the axes of these two rollers.

A further embodiment provides that for each axial rolling path, at least eight rollers or casters are supported rotatably in a circumferentially aligned position, in pairs, on a corresponding support frame, the said support frames being oscillatingly mounted around an axis parallel to the rotation axes of the rollers or casters and located intermediate to them, respectively, in the terminal zones of a carriage frame provided on opposite sides of a central support axis of said carriage frame.

In yet another embodiment, the said carriage frame is translatable relative to its radial position with respect to the arm's curvature axis.

A variant embodiment provides that the radial position adjustment elements of the carriage frame consist of the frame's support axis, which is eccentrically rotatable relative to the engagement housing in said carriage frame.

According to an additional characteristic, which can alternatively or in combination be provided with one or more of the previous embodiments and/or variant embodiments, two or more rollers or two or more casters for each of the two radial rolling paths can be provided, which casters or rollers are arranged one behind the other with respect to the angular extension of the corresponding radial rolling path. In this case, the said two or more rollers or the said two or more casters can be mounted on one or more carriages made according to one or more of the embodiments and variant embodiments described above.

The rolling paths are preferably made of relatively hard metal, such as steel or similar materials.

Furthermore, the rolling paths may be created in the form of metal strips or metal foils that are affixed by means of chemical/physical adhesion to the material of the arm.

Preferably, the fixing can take place by using double-sided adhesive materials in the form of tapes or double-sided adhesive pastes or coatings.

According to a variant embodiment and optionally, the double-sided adhesive material used for fixing can have a predetermined thickness, and in combination, the said material may exhibit a certain elastic deformability.

In one embodiment, the said rolling paths and the double-sided adhesive material are housed in a groove provided in the material of the projecting fins and/or in the side walls of the arm, the depth of which is such that, in the fixed condition, the said paths are flush with the surfaces of the corresponding fins and side walls of the arm.

Furthermore, the width of the rolling paths and the width of the housing grooves can be set such that the rolling paths adhere with their longitudinal side edges in contact with the longitudinal edges of said grooves.

In one embodiment, the arm has a rectangular central section with a predetermined axial dimension and a predetermined radial dimension with respect to the curvature axis, with the side walls oriented radially to the said curvature axis, while the projecting fins consist of axial extensions of the circumferential walls, that is, oriented in an axial direction with respect to the curvature axis of the arm, beyond the junction edges with the radial side walls of the arm itself.

The sliding of the arcuate arm relative to the slide support can take place either by means of motorised translation elements or by manual actuation by an operator.

In the case of motorised actuation, this can be implemented according to one or more of the translation systems currently known in the state of the art. Examples of these motorised translation elements include a drive belt connected at its ends to the arm and engaged on a motorised pulley attached to the support or base for executing movement in both directions of the arcuate arm; a translation system using a motorised pinion and rack assembly, where the motorised pinion is attached to the slide support and/or the base, and the rack is attached to the arcuate arm, or vice versa; or an assembly consisting of at least one motorised drive roller affixed to the base or support, which drive roller adheres with a predetermined friction force against the arcuate arm or vice versa. The aforementioned list is not exhaustive but merely provides some examples known in the state of the art, being possible that any type of drive system for translating the arm relative to the slide support may be provided in combination with one or more of the characteristics of the apparatus described in this document.

Although the preferred embodiment and the embodiments described with reference to the exemplary implementations shown in the drawings feature an arcuate arm with only one rolling path for each axial guide and/or for each radial guide, it is possible to provide an embodiment in which each axial guide and/or each radial guide has two or more rolling paths, respectively parallel and coaxial to each other or concentric with each other, with the slide support provided with at least one roller or two or more rollers in a row in the direction of the angular extension of said rolling paths, which two or more rollers may be mounted on carriages constructed according to one or more of the variant embodiments described above. Indeed, adapting the described embodiments to include two or more paths for each axial guide and/or each radial guide does not require substantial modifications to the described structural configurations, whether for the more generic or the more specific embodiments.

According to an additional aspect of the apparatus subject to this invention, an embodiment of said apparatus, which can be provided in combination with or separately from one or more of the above-described embodiments and variants, comprises a base with a support for an arcuate arm, preferably in the shape of a circular sector, also referred to as a "C"-shaped arm, which arcuate arm has two opposing ends spaced apart by a predetermined distance relative to a diametric plane parallel to the curvature axis of said arm, with an X-ray source supported at one end of the arm and directed towards the opposite end, along the propagation direction of the X-rays and being an X-ray detector supported at the opposite end of the arm, facing the X-ray source with a detection element for said X-rays, and wherein
one between said source or said detector is translatable along a rectilinear path in the direction of the opposite end of the arm, while the other between the said source or said detector is fixed in a stationary manner to the corresponding end of the arm, while, in correspondence with the said detector or the said source that is stationary-mounted on the corresponding end of the arm, a balancing mass is provided to compensate for the shift in the center of gravity upon the translation respectively of said source or said detector, which is movably coupled to the corresponding end of the arm, which mass is mounted translatably along a predetermined path and is movable by means of motorised actuators, with a control unit provided that detects the magnitude of the translational stroke of said source or said detector and operates said motorised actuator for the displacement of the balancing mass, executing a balancing stroke determined as a function of the detected magnitude of the translation stroke of the source or the detector.

In essence, in the said embodiment, one between the source or the detector is translatable relative to the end to which it is mounted, while the other between said source and said detector is fixed stationary at the corresponding end, and respectively to the source or to the detector fixed stationary there is also associated, on the corresponding end, a compensation mass which is translatable along said predetermined path.

In one embodiment, both the source and detector may be translatable essentially towards each other, with differing translation strokes between the source and detector, a dedicated motorised actuation unit being provided for the translation of the source and for the translation of the detector, while an integration mass is associated or associable with either the source or detector, to compensate for any mass difference between the source and detector or alternatively, a control element for the translation actuators is provided, which commands them to execute translation strokes of differing magnitudes for the source and detector, in order to maintain the arm's center of gravity in a stable position.

Various types of motorised actuators may be used.

A preferred, though non-limiting, embodiment provides that the mass and/or the source and/or the detector are mounted on a slide affixed to a linear actuator, the stroke of which is parallel to the intended translation stroke. A variant embodiment provides that the said slide is translation-coupled with a lead nut engaged on a threaded rod, with the threaded rod driven in rotation by a motor and the lead nut non-rotatably mounted on the threaded rod, but translatable axially along it. A specific embodiment may include the use of linear actuators of the so-called recirculating ball type.

Thanks to the translation of the source or detector, or both, optionally over differing translation strokes, it is possible to move the examination subject closer to or further from them, and to vary, in particular, both the field of view and the magnification factor obtained by the projection of the X-rays through the body under examination.

Compensation for the shift in the center of gravity position of the arcuate arm-source-detector assembly maintains the stresses on the slide support unchanged, preventing any center of gravity shifts from positioning it non-coincident with the C-arm's rotation axis, thus avoiding moments or torques perpendicular to the slide support and sliding guides of the arcuate arm, thereby reducing the likelihood of clearances arising between the guides and the slide support due to increased stresses on these components.

In particular, when the arcuate arm is of a lightweight construction, as described in the previous embodiments, and the contact between the arm's guides and slide support is limited to narrow contact bands and similarly narrow circumferential contact surfaces, the mechanical pressures generated between these contact surfaces, due to moments or angular forces acting on the arm resulting from the center of gravity shift, are considerable and, in addition to the wear of said contact surfaces, they may cause the detachment of the reinforcing profiles forming the rolling paths along the arm's guides and/or produce non-uniform wear of the reinforcement rings and rolling elements of the bearings forming the casters that cooperate with said rolling paths.

Moreover, a center of gravity shift, and thus an imbalance in the arcuate arm-X-ray source-detector assembly, results in greater effort required by an operator tasked with the manual actuation of the arcuate arm's translation. This can be not only inconvenient but also highly challenging, especially when the service personnel lack sufficient physical robustness.

Additionally, during manual movement of the arcuate arm/source/detector assembly, a center of gravity shift would cause a rotational movement of the arm itself, which could pose a hazard. Indeed, with manual actuation, an unbalanced arm, if left unsecured by an operator, might move unexpectedly, risking collision with the patient on the operating table. In other words, the arm must always be balanced; otherwise, it would be dangerous to operate it manually. In the current state of the art, unbalanced C-arms exist but are designed for motorised movement only and feature several safety mechanisms, including dual drive belts or, optionally, non-reversible transmissions, which prevent the arm's translation unless the motor is engaged.

It is therefore evident that there exists a functional synergy between the specific "lightweight" construction of the arm, according to one or more of the above-described variants, and one or more of the embodiments provided for compensating the center of gravity shift relative to the arcuate arm-source-detector system, despite the fact that the structural embodiments of the arm and the technical measures for compensating the center of gravity shift can be provided independently of each other, still delivering technical advantages compared to apparatuses known in the state of the art.

Further features are the subject of the dependent claims.

These and other features and advantages of the present invention will become more apparent from the following description of some exemplary embodiments illustrated in the attached drawings, wherein:
Fig. 1 shows a schematic side view of an embodiment of an apparatus for diagnostic radiographic imaging, particularly during interventions, the said apparatus featuring an arcuate arm for supporting at least one source and at least one detector.
Fig. 2 shows a cross-sectional view along a radial plane parallel to the curvature axis of the arcuate arm according to Fig. 1, where the arm's slide support is only partially illustrated.
Fig. 3 is a perspective view of a segment of the C-shaped arcuate arm according to Fig. 2, showing only the bearings forming the casters for the C-arm's slide support.
Figs. 4 and 5 show the linear translation systems of the source and/or detector and the center of gravity compensation mass, in a non-limiting embodiment in which only the detector is linearly translatable, while the source is fixed on the arm and the translatable compensation mass is associated with said X-ray source, respectively with the detector and compensation mass in their position of maximum separation from each other, and with the detector and compensation mass in their position of maximum proximity.
Fig. 6 displays an enlarged detail of the arm in the previous figures related to the support end of the detector.
Fig. 7 shows an enlarged detail of the arm from the previous figures regarding the support end of the X-ray source.
Figs. 8 and 9 show an axial view of an enlarged detail of the slide support zone, with the housing of said slide support omitted, showing only the rolling carriages on the corresponding side of the arcuate arm and a section along a radial plane of the guide fins.

Regarding the embodiment shown in the figures, this pertains to a radiological apparatus of a portable type, featuring a trolley component. However, said radiological apparatus can also be configured as a stationary device, i.e., non-portable. Additionally, the illustrated embodiment may be realized according to any configuration of the constituent parts shown in general terms.

With reference to the radiological apparatus, it comprises a base 1 mounted on or configured as a trolley 2. The base 1 forms the housing for one or more control units 3 of the apparatus, particularly for powering and controlling the X-ray source 4, as well as for controlling the detector 5. Furthermore, the base 1 can also house a processing unit 6 for the data received from the detector 5 in order to process one or more images from the data, store them, and/or display them on a display 7. The processing unit 6 and/or the one or more control units 3 are connected or connectable to human-machine communication interfaces denoted by 8 and can be of any known type for issuing messages from said units to human users and for receiving commands from human users. Non-limiting examples include visual display devices like display 7, keyboards, touchscreens, gesture detectors, vocal message generators, and/or vocal command input devices or other types of interfaces for bidirectional communication between humans and the apparatus. It is intended that box 8 represents any combination of one or more of these interface devices.

The control unit and/or processing unit may also be connected to or include a communication unit, common or dedicated, denoted as 9 and configured to allow communication between the apparatus, i.e., said control and/or processing units, and remote units for managing both the data collected during image acquisitions and for managing maintenance of the apparatus.

A slide support 10 for an arcuate arm, such as a "C"-shaped arm 11, is fixed to the base 1.

The slide support 10 and the arm, which will be described in greater detail below, are mounted on the base rotatably about an axis "CR," which preferably passes through the curvature axis "AC" of the arm 11 and is oriented substantially horizontally. A rotation unit, i.e. an actuation unit for said rotation movement, is indicated as 12 and is widely known in the state of the art in a variety of configurations from which a skilled technician can choose based on their needs.

At the opposing ends of the arcuate arm 11, the X-ray source 4 and the detector 5 are mounted. These can be realized according to one or more of the embodiments known in the state of the art and depend, in part, on the desired radiographic imaging acquisition methods.

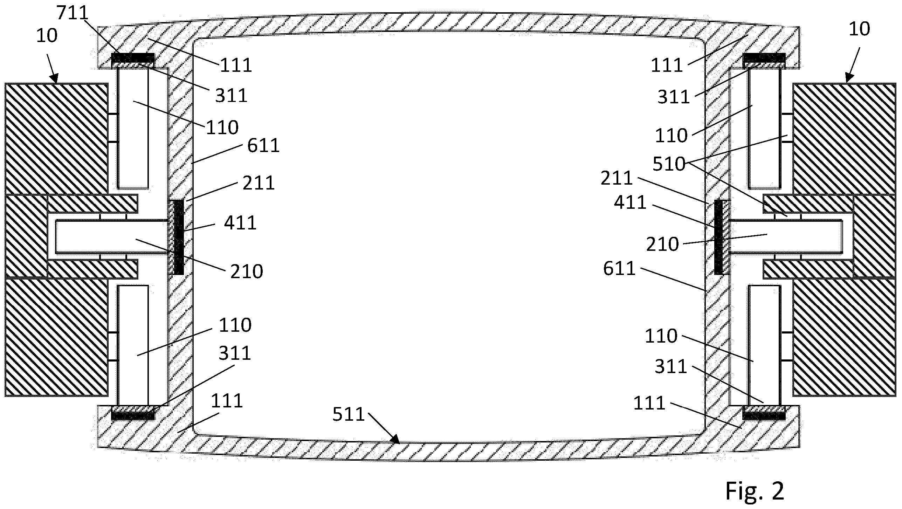

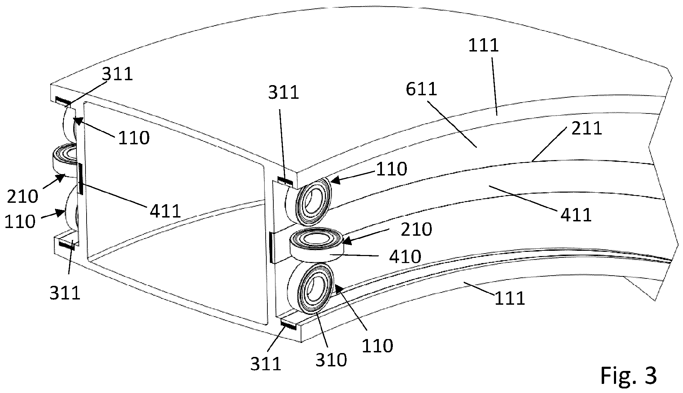

With reference to Figures 2 and 3, in one embodiment of the present invention, the arcuate arm 11 is constructed from a composite material comprising mineral and/or natural fibers embedded in a plastic materials matrix.

As for possible mineral fibers, it is possible to provide long fibers of various plastic polymers, such as nylon fibers or similar, glass fibers, rock fibers, or similar, as well as carbon fibers and/or combinations of said fibers.

Said fibers or combinations of fibers may be provided in woven form, such as a fabric or a non-woven fabric.

These fibers or combinations thereof are embedded in a material matrix, which may be plastic, a plastic blend, a resin, a resin blend, or even a metal.

Using such a material allows the arcuate arm to achieve substantial mechanical rigidity while simultaneously reducing weight compared to solutions that use metallic profiles. Reducing the weight of the arcuate arm 11 also enables a corresponding reduction in the base weight, as the counterbalanced weight of the arm is lessened, resulting in a significantly lighter and more easily maneuverable apparatus.

However, from a mechanical perspective, the composite material containing carbon fibers proves less resistant to surface wear caused by contact with the rotating or sliding elements that allow the translation of the arm within the slide support 10.

In the illustrated embodiment, the slide support 10 is provided with casters or rollers 110 210 that adhere with their rolling surfaces to rolling paths 311, 411 provided on rolling or sliding guides 111, 211 of the arcuate arm 11.

According to a first characteristic, said rolling or sliding guides are formed integrally with a central part 511 of the profile that constitutes the arcuate arm 11, specifically, on each side 611 of the arm 11 substantially oriented transversely to the curvature axis of the said arm 11, with a pair of fins 111 protruding beyond the corresponding side 611 and being coaxial with the curvature axis of the arm 11, parallel to each other and spaced apart by a certain distance.

A third sliding or friction guide is constituted for each side of the arm by the corresponding lateral wall 611 of the central body 511 of the arm 11 itself.

As is evident, an exemplary, non-limiting embodiment may provide that the said fins 111 are made as lateral extensions of the circumferential walls, both radially outer and radially inner, of the central body 411 of the arm 11, and these fins 111 have a predetermined projection relative to the corresponding lateral wall 511.

According to an additional feature, the fins forming said sliding or friction guides 111 are made with a thickness greater than at least that of the circumferential walls of the central body 511 of the profile constituting the arcuate arm 11.

In correspondence with the contact bands between the peripheral surfaces of the rollers 110, 210, the fins forming the circumferential sliding guides 111 and the lateral walls 611 of the profile body 511 have rolling or sliding tracks indicated by 311 and 411, which are made of hard material, specifically metallic, and are fixed to the corresponding fins and corresponding lateral walls on the side facing the rollers or casters 110, 210.

In one embodiment, the said rolling or sliding tracks 311, 411 are recessed inside grooves or coincident slots etched into the thickness of these guides 111, 611.

In one embodiment, the said rolling or sliding tracks 311, 411 are fixed by chemical/physical adhesion, for instance, through an adhesive application as shown by 711.

In one embodiment, the said rolling or sliding tracks 311 and 411 are in the form of flat tapes or metal sheets with a width corresponding to or slightly larger than the axial width of the peripheral contact surfaces of the rollers or casters and a thickness substantially smaller than the said width.

Furthermore, according to a feature, the depth of the grooves or slots is such that the surfaces of these rolling or sliding tracks 311, 411 extend substantially flush with the adjacent surface of the guides 111, 611 or are slightly protruding beyond the adjacent surfaces of the guides 111, 611.

In the illustrated embodiment, the two rolling or sliding guides 111 are circumferentially and coaxially oriented to the curvature axis of the arm 11 and to the central part 511, and the rolling or sliding tracks 311 are provided on or at least partially recessed within the sides of the said guides 111 that face each other, while the casters or rollers 110 are radially spaced apart from each other, with reference to the curvature axis of the arm, and in such a way that each of these rollers contacts the corresponding sliding track 311 with a certain force. The radial arrangement of these casters and rollers relative to each other is configured such that the arcuate arm is held in position between them without exhibiting any radial clearance, while allowing the arm to slide without excessive resistance.

In one embodiment, the said casters or rollers are preferably constituted by bearings having an inner race 310 that is radially keyed to a spindle 510 carried by the sliding support 10, and an outer race 410 that adheres against the corresponding rolling or sliding track 311, being the bearing rolling bodies provided between the two races.

Any type of bearings, such as ball bearings or those with cylindrical, elliptical, or biconical rolling bodies, or other types of bearings, may be used.

With reference to the rolling or sliding tracks 411, these are provided in the two opposite lateral walls 611 of the profile that constitutes the central body 511 of the arcuate arm. The rolling or sliding tracks 411 are coaxial and radially aligned with reference to the curvature axis of the arm 11 and are manufactured according to one or more of the embodiments previously described for the circumferential rolling or sliding tracks 311.

Furthermore, the rollers or casters 210 are also made in the form of bearings, and they are mounted with the inner race on a spindle 510 that is radially oriented. The axial distance between the spindles on which the rollers or casters 210 are mounted, combined with their external radius, is such that the rollers or casters 210 adhere to the corresponding rolling or sliding track 411 with a preset force, holding the arcuate arm in position without axial play while allowing it to slide along its circumferential extension.

According to yet another characteristic, not illustrated in detail, it is possible to provide that the supports of the spindles 410 for the rollers or casters 110, 210 are made in such a way as to allow at least the radial position of the spindles to be adjusted to regulate the support force against the corresponding rolling or sliding track.

According to a possible variant, while Figures 2 and 3 show only one pair of rollers or wheels 110 cooperating with the circumferential rolling or sliding tracks 311 and a pair of opposite rollers or casters 210 cooperating with the lateral rolling or sliding tracks 411, in these additional possible variants, multiple pairs of the aforementioned rollers or casters may be provided which are arranged at a certain distance from each other in the direction of the arm's sliding motion, with each adjacent pair generating additional support points for the arm 11 within the sliding support 10.

In one embodiment, each pair of casters or rollers, or two or more pairs of them, or individual casters or rollers, that is their corresponding axes, are supported on trolleys that are configured like levers or with functions corresponding to lever mechanisms oscillating around at least one or more axes parallel to the curvature axis of the arcuate arm 11.

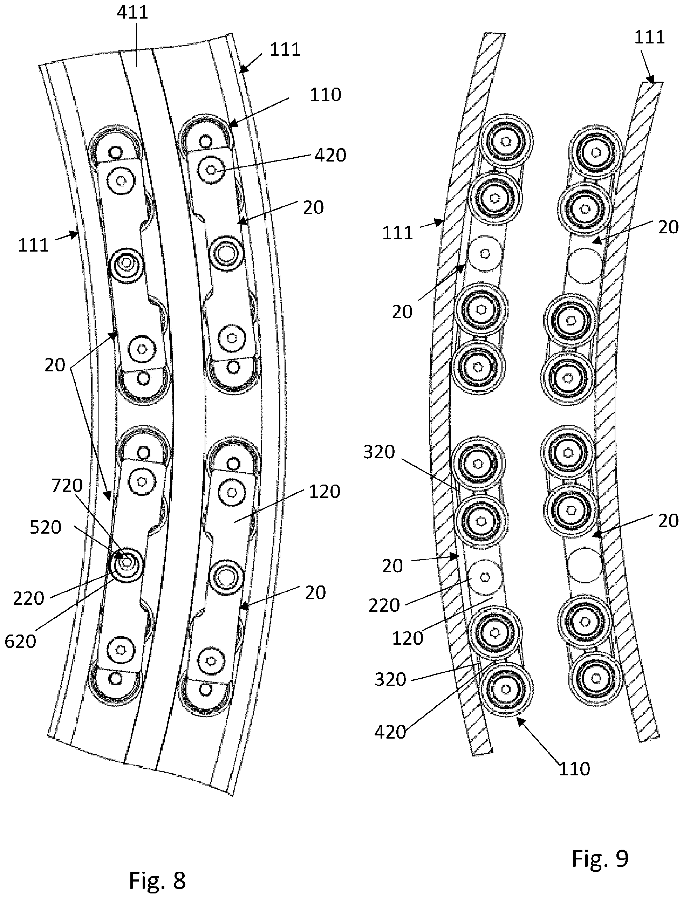

Without the illustrations in Figures 8 and 9 and the descriptions referenced thereto being considered a limitation on the general concept presented above, the embodiment of these trolleys, indicated globally as 20, includes four trolleys 20 for each side of the arcuate arm 11, two of which are respectively arranged in series in the circumferential direction of the rolling tracks and carry rollers or casters 110 that cooperate with one of the two opposed axial rolling tracks 311 on the corresponding side of the arcuate arm 11.

In a non-limiting embodiment, each of these trolleys 20 comprises a trolley frame 120 that is mounted on a support axle 220 oriented parallel to the curvature axis of the arcuate arm 11. The trolley frame 120 includes two side members oriented transversely to the circular path of the guides 111 and that, at their opposite ends, respectively support an oscillating frame 320 supporting two rollers or two casters 110. These rollers or casters 110 are mounted rotatable around an axis parallel to the curvature axis of the arcuate arm 11, in the area of the two opposite ends of the oscillating support frame 320, while each oscillating frame 320 is supported oscillating around an oscillation axis 420 intermediate to the rotation axes of the respective rollers or casters 110.

The rollers or casters 110 may be made according to one or more of the previously described variants.

According to an advantageous feature, each trolley 20 has adjusting mechanisms 520 for the radial position relative to the corresponding rolling track 311.

In a non-limiting embodiment, the trolley frame 120 has cylindrical seats 620 for engagement with the support axle 220 itself, which axle has a corresponding cylindrical shape, at least for a segment coinciding with the corresponding seat 620, with at least the said segment fixed to rotate around an eccentric axis relative to the symmetry axis of the cylindrical section of the same and/or of the corresponding seat 620, and having rotation elements 720 for rotation and locking/unlocking in the angular position of the said cylindrical segments and/or the entire cylindrical support axis around the said eccentric rotation axis.

With reference to a further additional characteristic that may be provided in combination or alternatively to the above-described embodiments, it is possible to provide a balance support trolley that carries two or more rolling casters 210 intended to cooperate with the corresponding radial rolling tracks 411.

Similarly to what is provided for the axial rolling tracks 311, for each radial rolling track 411 on each side of the arcuate arm 11, a single trolley 20, two or more trolleys 20 aligned with each other in the direction of the angular extension of the corresponding track, may be provided.

Likewise, each trolley 20 can be made in accordance with one or more of the previously described construction variants with reference to Figures 8 and 9.

A construction variant may provide that the trolley includes a frame with arcuate lateral side members that are coaxial with the curvature radius of the radial sliding track 411, or that it is composed of a polygonal frame allowing the placement of the roller axes in the radial direction relative to the curvature axis of the radial rolling track, and/or that the axes of these rollers are supported in the corresponding trolley so as to be radial with respect to the said curvature axis of the radial rolling track 411.

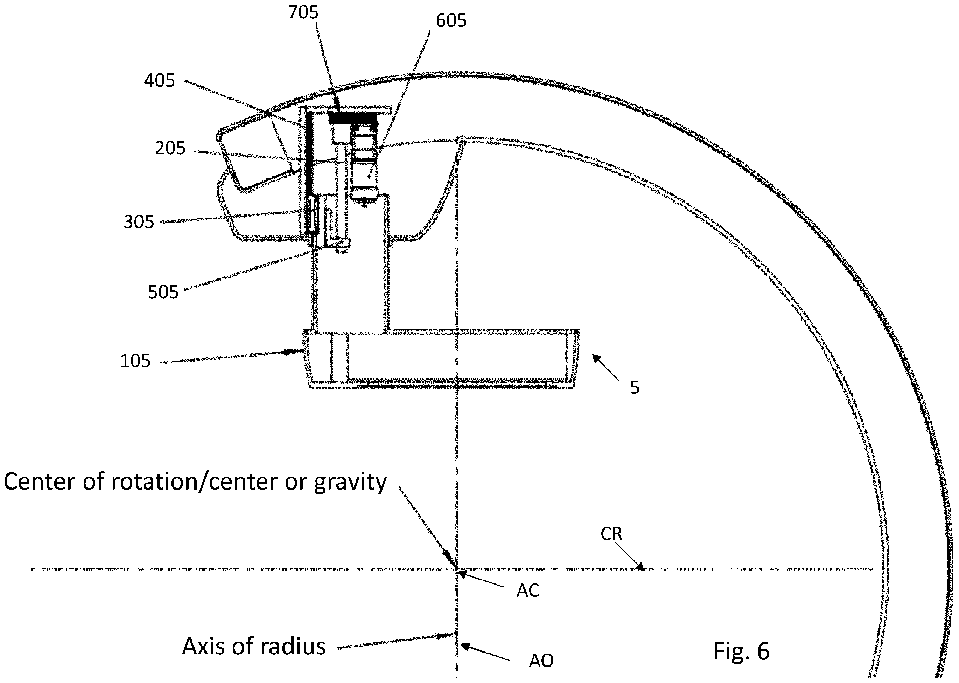

With reference to Figure 6, this relates to an embodiment that may be provided in combination or separately from the above-described embodiments, in which the upper end of the arcuate arm 11 carries an X-ray detector, referenced as 5, which can be made according to any of the currently known technologies.

The sensor, for example, a CCD or other type of sensor 105, is positioned with its optical axis substantially aligned with the optical axis of propagation of the X-ray beam emitted from the X-ray source 4.

A support for the sensor 105 is movable essentially parallel to the direction of the said optical axis "AO" and includes a support slide 305 to which at least the sensor 105 of the detector is attached, the slide 305 being mounted to slide along a guide 405 and in a direction parallel to the optical axis "OA", being the said slide 305 actuated to move bidirectionally via an assembly that includes a lead nut 505 firmly attached to the slide 305 without rotation and engaged with a threaded rod 205, which is driven in rotation by a motor 605 through a transmission gear set 705 for transferring motor movement to the said threaded rod.

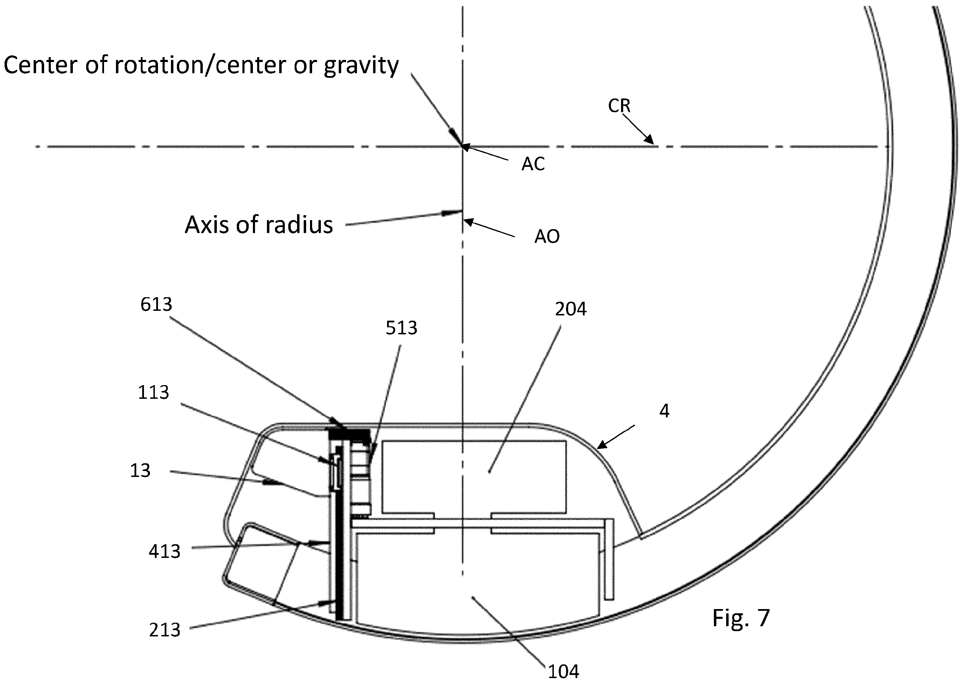

Since moving the sensor toward and away from the center of rotation of the arcuate arm, essentially coinciding with its axis of curvature, leads to a shift in the center of gravity of the arcuate arm system 11, the detector 5, and the X-ray source 4, as shown in Figure 7, a counterweight 13 is provided at the opposite end of the arm from the detector 5 that carries the said X-ray source 4, and this counterweight 13 is mounted to move in a direction parallel to the direction of the said optical propagation axis OA of the X-ray beam.

The counterweight 13 is movable through means substantially similar to those previously described for moving the sensor 105 of the detector 5.

Specifically, a slide 113 is mounted to slide along a guide 213. The guide is oriented parallel to the direction of the aforementioned optical axis OA, while the movement of the slide along the guide is achieved through a linear actuator consisting of a lead nut 313 fixed non-rotatably to the slide 113 and engaged on a threaded rod 413, which is driven to rotate about its own axis by a motor 513 and a transmission gear set 613.

The X-ray source includes an X-ray generating tube indicated as 104 and a collimator 204 for forming and/or confining the X-ray beam projected toward the detector.

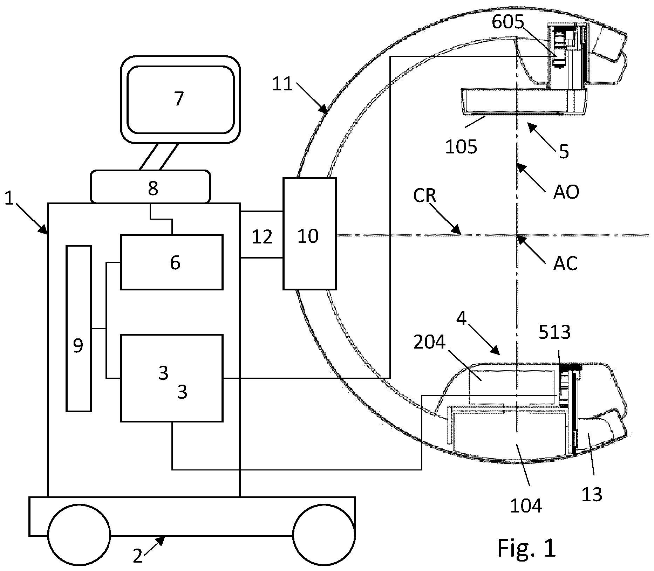

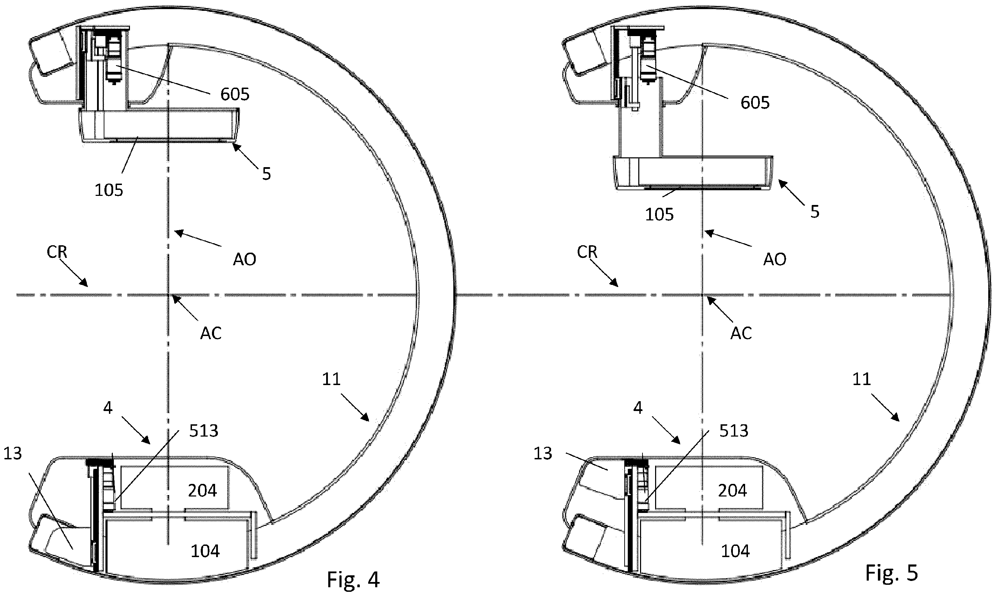

As is evident from Figures 1, 4, and 5, the motors 605 and 513, respectively intended to control the translation of the slide 305 carrying the sensor 105 and the slide 113 carrying the counterweight 13, are controlled by the control unit 3, which is configured, either by an algorithm encoded in control software or by a hardware setup, to command a translation of the counterweight 13 in response to a translation of the sensor 105 of the detector 5, such that the center of gravity of the arcuate arm system 11, detector 5, and X-ray source 4, including the counterweight 13, remains consistently coincident with the center of rotation CR, as indicated in Figures 1 and 4 to 7.

Thanks to this feature, stresses on the sliding support and the guides of the arcuate arm 11, due to torques or angular moments caused by the movement of the detector mass 5, are avoided, while also enabling adjustment of the distance between the sensor 105 of the detector and the X-ray source 4, i.e. the collimator, to modify the field of view (also known as FOV) and/or the magnification factor of the projection on the detector 5.

This ensures that forces resulting from imbalance of the arm relative to the sliding support do not act on the arcuate arm made from the relatively lightweight carbon fiber construction and on the rolling or sliding tracks, chemically and physically adhered to the profile constituting the said arm, which could cause damage or rapid wear to the sliding support and guide combination of the arcuate arm.

In yet another embodiment, the movement of the counterweight may be manually or automatically controlled.

In the case of manual translation of the arcuate arm, i.e., a translation where the force is exerted by an operator, translating the counterbalance allows the arm to remain balanced at its center of gravity, thus avoiding the need for the operator to apply additional physical effort to compensate for angular torques that would arise from the offset of the center of gravity relative to the support, enabling manual movement of the arcuate arm along the sliding support even by personnel who may not be physically robust.

This advantage is further beneficial in the case of manual rotation of the arcuate arm around the radial rotation axis, which is transverse to the spacing direction between the two ends of the arcuate arm itself.

| | |
|---|---|
| Base | 1 |
| Trolley | 2 |
| Control unit | 3 |
| X-ray source | 4 |
| Detector | 5 |
| Processing unit | 6 |
| Display | 7 |
| Human-machine interfaces | 8 |
| Communication unit | 9 |
| Sliding support | 10 |
| Arcuate arm | 11 |
| Rotation unit | 12 |
| Rollers or casters | 110, 210 |
| Rolling or sliding guide | 111, 211 |
| Rolling or sliding tracks | 311, 411 |
| Central part | 511 |
| Lateral wall | 611 |
| Coating or double-sided adhesive | 711 |
| Inner bearing race | 310 |
| Spindle | 510 |
| Outer bearing race | 410 |
| Detector sensor | 105 |
| Slider | 305 |
| Guide | 405 |
| Lead nut | 505 |
| Threaded rod | 205 |
| Motor | 605 |
| Gear assembly | 705 |
| Balancing mass | 13 |
| Slide | 113 |
| Guide | 213 |
| Threaded rod | 413 |
| Motor | 513 |
| Gear assembly | 613 |
| X-ray generator tube | 104 |
| Collimator | 204 |
| Trolley | 20 |
| Trolley frame | 120 |
| Support axis | 220 |
| Oscillating frame | 320 |
| Oscillation axis | 420 |
| Adjustment components | 520 |
| Cylindrical seats | 620 |
| Rotation elements | 720 |

## Claims

1. Apparatus for image acquisition with an arc-shaped or C-shaped support arm comprising:
- a base (1) and a slide support (10) for an arcuate arm (11), preferably in the form of a circular sector, said arcuate arm (11) being coupled to or having slide guides (111, 211) along its circumferential extension, said slide guides cooperating with rotatable supports (110, 210) rotatably mounted in said slide support (10) of said arcuate arm, being optionally provided motorized actuating device for said sliding,
wherein said arm (11) is entirely made of an arcuate profile in composite material comprising one or more reinforcing fibers, preferably one or more mineral fibers, particularly carbon fibers, and an embedding matrix of said fibers, said profile constituting the body of the arm and having one or more guiding surfaces (111, 211) with axial and/or radial orientation, i.e., parallel and/or perpendicular to the axis of curvature of the arm (11) itself, and each guiding surface having one or more rolling paths (311, 411) of at least one corresponding roller or at least one corresponding caster (110, 210), which are respectively formed of continuous metallic profiles that are fixed in a position coinciding with the paths of the peripheral contact surfaces of said rollers or said casters (110, 210) and are directed towards said peripheral contact surfaces of said rollers or said casters along said guiding surfaces.

2. Apparatus according to claim 1, wherein the guiding surfaces (111) are constituted by a pair of fins projecting outwardly in a direction parallel to the axis of curvature of the arm, to a predetermined extent from each of the two opposite side walls (611) of the profile, which are oriented in a direction perpendicular to the axis of curvature of said arm (11) and are parallel i.e. concentric with respect to each other, having said rolling paths (311) a circumferential extension and arranged on the mutually facing sides of said fins (111), with at least one pair of rollers or casters (110) being provided on each side of the arm, the rollers of each pair being in contact respectively with one of the two rolling paths (311) on the mutually facing sides of said fins (111).

3. Apparatus according to claims 1 or 2, wherein at least one third rolling path (411) with radial orientation or two or more concentric rolling paths are provided, which is/are coaxial with respect to the axial rolling path(s) i.e. with a circumferential course (311), and said radial rolling path(s) is/are provided on each of the two opposing side walls (611) of the arm (11) along the band (211) of said side walls (611) which is radially bounded by said projecting fins (111) on the corresponding side, while the sliding support (10) is provided with at least one roller or at least one caster (210) that is mounted on the sliding support (10) of the arm (11) itself in a position such as to adhere respectively against one of said radial, lateral tracks (411).

4. Apparatus according to one or more of the preceding claims, wherein the rollers or the casters consist of bearings whose inner race (310) is keyed to a spindle (510) coaxial to the axis of rotation and whose outer race (410) adheres with its outer surface against the corresponding rolling path (311, 411) .

5. Apparatus according to one or more of the preceding claims, wherein the rolling paths (311, 411) are made of a hard material, preferably metal, such as steel or similar.

6. Apparatus according to one or more of the preceding claims, wherein said rollers or said casters are provided along the radially outer shell wall and in contact with the rolling paths, with an outer covering of elastically deformable material having a predetermined value of elastic deformability, particularly of plastic material.

7. Apparatus according to one or more of the preceding claims, wherein two or more rollers or two or more casters (110, 210) cooperating with one or more axial rolling paths (311) or one or more radial rolling paths (411) are mounted on at least one carriage (20) or on a succession of carriages (20) configured as a rocker arm.

8. Apparatus according to one or more of the preceding claims, wherein the rolling paths (311, 411) may be made in the form of metal strips or metal foils fixed by chemical/physical adhesion to the material of the arm (11), optionally housed in a groove provided in the material of the slide guides (111, 211), the depth of which is such that, when fixed, said paths extend flush with the surfaces of the corresponding slide guides (111, 211).

9. Apparatus according to one or more of the preceding claims, wherein the arm (11) consists of a profile with a cross-section having a rectangular central part (511) with a predetermined dimension in the axial direction and a predetermined dimension in the radial direction with respect to the axis of curvature, and with side walls (611) oriented radially with respect to said axis of curvature, while the projecting fins (111) consist of axial extensions of the circumferential walls, i.e., oriented in the axial direction with respect to the axis of curvature of the arm, and extend beyond the joint edges with the radial side walls of the arm itself.

10. Apparatus according to one or more of the preceding claims, wherein the arcuate arm (11) has two ends opposite each other and spaced apart by a predetermined distance with respect to a diametric plane parallel to the axis of curvature of said arm, an X-ray source (4) being supported at one end of said arm and directed toward the opposite end in the direction of X-ray propagation, and an X-ray detector (5) being supported at the opposite end of the arm (11) and facing the X-ray source (4) with a detection sensor (105) for said X-rays,
wherein
one of said source (4) or said detector (5) is translatable along a straight path toward the respective opposite end of the arm (11), while the other, either the detector (5) or the source (4), is fixed stationary to the corresponding end of the arm, and at the position of the said stationary coupled detector or source, a balancing mass (13) is provided to compensate for the displacement of the center of gravity during the translation of said source (4) or detector (5), that is movably coupled to the corresponding end, which mass (13) is mounted in a translatable manner along a predetermined path and is movable by means of motorized actuators (513), being provided a control unit (3) to detect the magnitude of the translation stroke of said source (4) or detector (5) and to actuate said motorized actuator (513) for moving the balancing mass (13) to perform a balancing stroke determined according to the detected magnitude of the translation stroke of the source (4) or detector (5) .

11. Radiographic apparatus with an arc-shaped or C-shaped support arm comprising a base with a support for an arcuate arm, preferably in the form of a circular sector, also referred to as a "C"-shaped arm, wherein said arcuate arm has two ends opposite each other and spaced by a predetermined distance with respect to a diametric plane parallel to the axis of curvature of said arm, an X-ray source being supported at one end of said arm and directed toward the opposite end in the direction of X-ray propagation, and an X-ray detector being supported at the opposite end of the arm and facing the X-ray source with an element for detecting said X-rays,
wherein
said source or said detector are translatable along a straight path toward the opposite end of the arm, while at the location of the detector or source, which is stationary coupled to the corresponding end of the arm, a balancing mass is provided to compensate for the displacement of the center of gravity during the translation of said source and/or said detector, which mass is mounted in a translatable manner along a predetermined path and is movable by means of motorized actuators, being provided a control unit that detects the magnitude of the translation stroke of said source or detector and actuates said motorized actuator for moving the balancing mass to perform a balancing stroke determined as a function of the detected magnitude of the translation stroke of the source or detector

12. Apparatus according to one or more of the preceding claims, wherein said source (4) or said detector (5) can be translatable substantially toward each other, with a different translation stroke, both for the source and the detector, a dedicated motorized actuator unit being provided for the translation of the source and for the translation of the detector, while an integration mass is or can be associated with the source or detector to compensate for any mass difference between the source and the detector, or alternatively, a control unit for the translation actuators is provided to command them to perform a translation stroke of different magnitudes for the source and detector to maintain the center of gravity of the arm stable.
